# EUROPÄISCHE PATENTANMELDUNG

(11) **EP 1 310 294 A1**
(43) Veröffentlichungstag der Anmeldung: **14.05.2003**
(21) Anmeldenummer: 02024338.2
(22) Anmeldetag: 02.11.2002
(51) Int. Cl.: B01J 4/00, B01J 19/26

(54) **Behälter ausgestattet mit einem Düsenkörper**

(30) Priorität: 12.11.2001 DE 10155565
(71) Anmelder: InfraServ GmbH & Co. Höchst KG, 65926 Frankfurt am Main (DE)
(72) Erfinder: Simon, Klaus-Jürgen, 65779 Kelkheim (DE); Itter, Klaus, 61184 Karben (DE)
(74) Vertreter: Ackermann, Joachim, Dr.

(57) **Zusammenfassung**

Beschrieben wird ein Behälter, der mindestens eine Eindüsungs- und/oder Entgasungsvorrichtung aufweist. Die Düsenbohrungen (4) sind dabei derart auf dem Düsenkörper (3) angebracht, dass zumindest einige davon sowohl in den Innenraum (1) als auch in der Behälterwand (2) münden.

Mit der beschriebenen Anordnung lassen sich Flüssigkeiten in den Behälter eindüsen, ohne dass Verwirbelungen von Luft und Flüssigkeit auftreten. Ein separater Entlüftungsstutzen kann entfallen.

## Beschreibung

Die vorliegende Erfindung betrifft einen Behälter mit einer Vorrichtung zum Eindüsen in und/oder Entgasen sowie dessen Verwendung in der Steriltechnik.

Behälter, die unter sterilen Bedingungen betrieben werden können, sind in vielen Gebieten der Technik, insbesondere in der pharmazeutischen und der chemischen Industrie üblich. In der Regel werden diese Behälter vor jedem Reaktionsansatz gereinigt und anschließend für die folgende Reaktion mit zu verarbeitenden Komponenten, wie Reaktanten, befüllt. Das Zuführen von Reinigungsflüssigkeiten bzw. von Komponenten kann durch Eindüsen erfolgen. In der Regel kommt es dabei zu Verwirbelungen von Luft und Flüssigkeit im Behälterinnern, so dass nach dem Befüllen eine Entlüftung des Innenraums über eine separate Entlüftungsvorrichtung notwendig ist.

Auch sind in vielen Fällen die Düsen dergestalt angeordnet, dass Toträume entstehen, die dem direkten Zugang von Reinigungsflüssigkeiten entzogen sind.

Die vorliegende Erfindung betrifft einen Behälter, der insbesondere zum Einsatz in der Steriltechnik geeignet ist, und der in einfacher Weise befüllt bzw. gereinigt und/oder entgast werden kann.

Die vorliegende Erfindung betrifft einen Behälter enthaltend mindestens eine Eindüsungs- und/oder Entgasungsvorrichtung, die an der dem Innenraum (1) zugewandten Seite der Behälterwand (2) angebracht ist und die mindestens eine, vorzugsweise mehrere in einem Düsenkörper (3) angebrachte Düsenbohrungen (4) aufweist, wobei der Düsenkörper (3) in die Behälterwand (2) hineinragt und zumindest eine, vorzugsweise mehrere der Düsenbohrungen derart im Düsenkörper (3) angebracht sind, dass sie sowohl in den Innenraum (1) als auch in der Behälterwand (1) münden.

Der erfindungsgemäße Behälter kann beliebige Ausgestaltungen haben. Üblicherweise weist der Behälter mindestens einen Deckel auf, der sich an beliebigen Stellen des Behälters befinden kann, vorzugsweise auf dessen Oberseite und/oder am Boden. Beispiele für erfindungsgemäße Behälter sind in der oben beschriebenen Weise ausgerüstete Rührkessel, insbesondere jedoch Drucknutschen und Chromatographiesäulen.

Ferner kann der Behälter Apparaturen aufweisen, die für die vorgesehene Betriebsweise erforderlich sind, beispielsweise Thermometer, Rührer, Sensoren, wie Füllstandsanzeiger, oder Zu- und Ableitungen von Edukten, Produkten oder Schutzgasen.

Der Düsenkörper (3) kann beliebige Formen aufweisen, beispielsweise einen rotationssymmetrischen aber auch rechteckigen Querschnitt. Bevorzugt weist der Düsenkörper (3) die Form einer Halbkugel auf.

Der Düsenkörper (3) kann auf beliebige Weise im Behälterinnenraum (1) angebracht sein, solange das Erfordernis erfüllt ist, dass zumindest einige der Düsenbohrungen sowohl in den Behälterinnenraum (1) als auch in der Behälterwand (4) münden. Der Düsenkörper kann im Behälterinnenraum rotierbar, vorzugsweise jedoch feststehend angebracht sein.

So kann der Düsenkörper (3) direkt auf der Behälterwand (4) befestigt sein und ist mit einer durch diese Behälterwand (4) führenden Zuleitung (5) verbunden. In einer bevorzugten Ausführungsform bildet der Düsenkörper (3) das eine Ende einer Zuleitung (5). Diese wiederum ist mittels eines Flansches (6) mit dem Behälter verbunden.

Der Düsenkörper (3) kann innerhalb des Behälters an verschiedensten Positionen angebracht sein. In einer bevorzugten Ausführungsform ist der Düsenkörper (3) am oberen Scheitelpunkt des Behälters angebracht.

Der Düsenkörper (3) kann die Düsenbohrungen (4) in beliebiger Anordnung aufweisen, solange das Erfordernis erfüllt ist, dass zumindest eine der Düsenbohrungen sowohl in den Behälterinnenraum (1) als auch in der Behälterwand (4) münden.

In einer bevorzugten Ausführungsform sind die Düsenbohrungen (4) in Form mehrerer, vorzugsweise parallel verlaufender Lochreihen im Düsenkörper (3) angebracht. Ganz bevorzugt sind alle Düsenbohrungen (4) einer der Lochreihen derart im Düsenkörper (3) angebracht, dass sie sowohl in den Behälterinnenraum (1) als auch an der Behälterwand (4) münden.

Durch die Düse können dem Behälter beliebige Agenzien und zu beliebigen Zwecken zugeführt werden, beispielsweise Flüssigkeiten, gegebenenfalls auch in Kombination mit Feststoffen, z.B. in der Form von Suspensionen, oder auch Gase. Vorzugsweise werden Flüssigkeiten eingedüst.

In einer weiteren Ausgestaltung kann die Düse als Entlüftungs- bzw. Entgasungsvorrichtung dienen.

So lässt sich die am oberen Scheitelpunkt angebrachte erfindungsgemäßen Vorrichtung zur vollständigen Entgasung des Behälters einsetzen, indem Flüssigkeit z.B. über den Behälterboden eingeleitet wird und das sich darüber befindliche Gas durch die erfindungsgemäße Vorrichtung entweichen kann. Durch die Anordnung der Düsenöffnungen ist eine vollständige Entgasung des Behälters möglich.

In einer bevorzugten Ausführungsform betrifft die Erfindung eine Drucknutsche oder eine Chromatographiesäule als Behälter. Von den Chromatographiesäulen sind insbesondere solche bevorzugt umfassend einen zylindrischen Säulenkörper, einen Boden, einen in Richtung der Zylinderlängsachse beweglichen, den zylindrischen Säulenkörper nach oben abdichtenden Deckel, eine im zylindrischen Säulenkörper angebrachte Auslassvorrichtung für das Füllgut und erfindungsgemäß angeordnete Düsenkörper als Aufschlämmeinheiten, die Flüssigkeit auf die Oberfläche des Füllguts eindüsen können, wobei der zylindrische Säulenkörper um eine senkrecht zur Zylinderlängsachse verlaufende Achse drehbar gelagert ist.

Bei der Auslassvorrichtung handelt es sich vorzugsweise um eine totraumarme bzw. -freie Vorrichtung.

Bevorzugte Ausgestaltungen dieser Auslassvorrichtung umfassen
a) ein Kugelgehäuse, das an der verschließbaren Öffnung mit dem Behälter verbunden ist
b) eine Absperrkugel, und
c) Mittel, um die Absperrkugel aus einer Schließposition in eine Öffnungsposition oder wieder zurück zu bewegen, wobei
   das Kugelgehäuse derart ausgestaltet ist, daß es bündig mit der
   Behälterinnenseite abschließt
   oder
d) ein Schiebergehäuse, das an der verschließbaren Öffnung mit dem Behälter verbunden ist
e) einen Schieber, und
f) Mittel, um den Schieber aus einer Schließposition in eine Öffnungsposition oder wieder zurück zu bewegen, wobei
das Schiebergehäuse derart ausgestaltet ist, daß es bündig mit der Behälterinnenseite abschließt.

Ein weiterer Vorteil der erfindungsgemäßen Behälters ist darin zu sehen, dass dieser gereinigt werden kann, ohne das eine Öffnung notwendig ist. Infolge des fehlenden oder des geringen Totraumes ist es ohne weiteres möglich, den Behälter von dessen Innenraum her mechanisch zu reinigen, beispielsweise mit mechanischen Mitteln oder durch den Einsatz von Wasserstrahlen. Ablagerungen und Keimbildungen an den Übergangsstellen können so vermieden werden, insbesondere wenn die mechanische Reinigung mit einer nachgeschalteten Sterilisation verbunden wird .

Der Vorteil der erfindungsgemäßen Behälter ist insbesondere darin zu sehen, daß zusätzliche Entlüftungsschritte beim kostenintensiven Reinigen bzw. bei der Beschicktung von Drucknutschen oder von Säulen mit aufzutrennendem Material, insbesondere bei Drucknutschen oder Säulen mit großen Durchmessern, beispielsweise von mehr als 600 mm und einem Bettvolumen von mehr als 500 I, entfallen kann.

Bevorzugt weist der erfindungsgemäße Behälter keinen zusätzlichen Entlüftungsstutzen auf.

Die Materialauswahl von Behälter und Eindüsungsvorrichtung hängt von dem ins Auge gefaßten Verwendungszweck ab. Als Materialien kommen Metalle aber auch Kunststoffe in Frage.

Die nachfolgenden Figur stellen beispielhaft eine Ausführung der vorliegenden Erfindung dar. Eine Beschränkung ist dadurch nicht beabsichtigt.

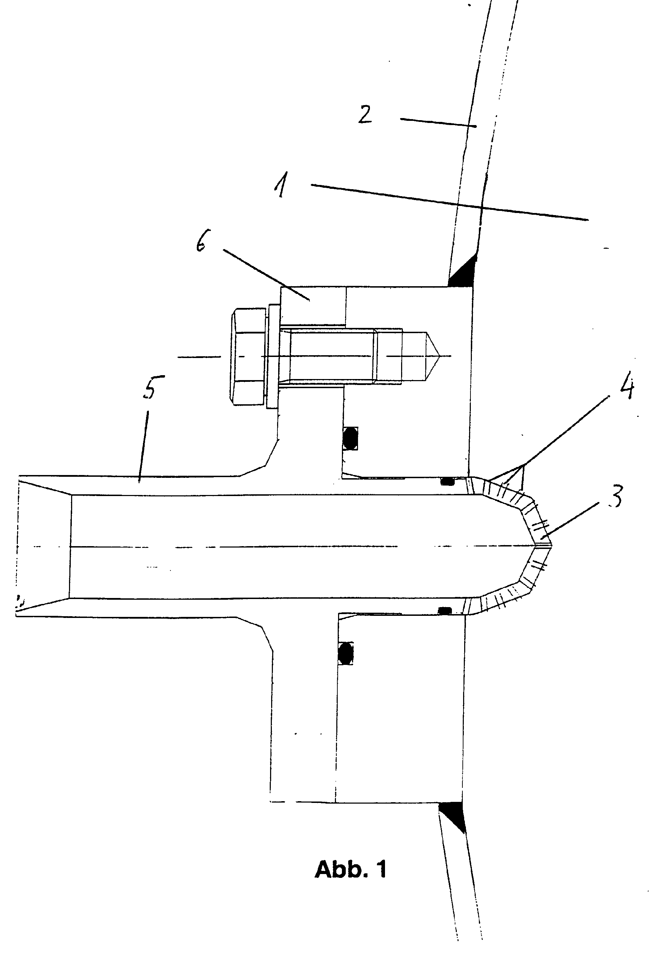

Die Figur zeigt eine Ausgestaltung einer Eindüsungsvorrichtung, die an einem Behälter angebracht ist.

In der Figur ist ein erfindungsgemäßer Behälter dargestellt. Die Figur zeigt eine Eindüsungsvorrichtung für Flüssigkeiten, die an der dem Innenraum (1) zugewandten Seite der Behälterwand (2) angebracht ist. Die Eindüsungsvorrichtung besteht aus einem Düsenkörper (3), der mehreren Düsenbohrungen (4) aufweist. In der dargestellten Ausführungsform weist der Düsenkörper (3) eine halbkugelförmige Gestalt auf. Der Düsenkörper (3) ist derart angebracht, daß er in die Behälterwand (2) hineinragt. Die Düsenbohrungen (4) sind in der Form von parallel verlaufenden Reihen angebracht (nicht dargestellt) und sind derart auf dem Düsenkörper (3) angeordnet, dass nach dessen Einbau in den Behälter die Düsenbohrungen (4) der untersten Reihe sowohl in den Innenraum (1) als auch in der Behälterwand (1) münden.

Der Düsenkörper befindet sich auf dem Ende einer Zuleitung (5), die mittels eines Blockflanschs (6) an dem Behälter mittels Schrauben befestigt ist.

Die erfindungsgemäßen Behälter kommen vorzugsweise bei der Herstellung oder Verarbeitung von sterilisierbaren Produkten, insbesondere von pharmazeutischen Produkten zum Einsatz.

Die Erfindung betrifft daher auch die Verwendung der in den Behälter angebrachten Eindüsungsvorrichtung zum Eindusen von Flüssigkeiten, insbesondere von Reinigungsflüssigkeiten oder von Reaktanten oder von zu trennenden Stoffgemischen.

Besonders bevorzugt werden sterilisierbare Behälter eingesetzt.

## Patentansprüche

1. Behälter enthaltend mindestens eine Eindüsungs- und/oder Entgasungsvorrichtung, die an der dem Innenraum (1) zugewandten Seite der Behälterwand (2) angebracht ist und die mindestens eine in einem Düsenkörper (3) angebrachte Düsenbohrungen (4) aufweist, wobei der Düsenkörper (3) in die Behälterwand (2) hineinragt und zumindest eine der Düsenbohrungen derart im Düsenkörper (3) angebracht ist, dass sie sowohi in den innenraum (1) als auch in der Behälterwand (1) mündet.

2. Behälter nach Anspruch 1, **dadurch gekennzeichnet, dass** die Eindüsungsund/oder Entgasungsvorrichtung mehrere in einem Düsenkörper (3) angebrachte Düsenbohrungen (4) aufweist, von denen zumindest einige sowohl in den Innenraum (1) als auch in der Behälterwand (1) münden.

3. Behälter nach Anspruch 1, **dadurch gekennzeichnet, dass** der Düsenkörper (3) die Form einer Halbkugel aufweist.

4. Behälter nach Anspruch 1, **dadurch gekennzeichnet, dass** der Düsenkörper (3) das eine Ende einer Zuleitung und/oder Ableitung (5) bildet, die mittels eines Flansches (6) mit dem Behälter verbunden ist.

5. Behälter nach Anspruch 1, **dadurch gekennzeichnet, dass** der Düsenkörper (3) am oberen Scheitelpunkt des Behälters angebracht ist.

6. Behälter nach Anspruch 1, **dadurch gekennzeichnet, dass** die Düsenbohrungen (4) in Form mehrerer, vorzugsweise parallel verlaufender Lochreihen im Düsenkörper (3) angebracht sind, wobei alle Düsenbohrungen (4) einer der Lochreihen derart im Düsenkörper (3) angebracht sind, dass sie sowohl in den Innenraum (1) als auch in der Behälterwand (2) münden.

7. Behälter nach Anspruch 1, **dadurch gekennzeichnet, dass** es sich um eine Drucknutsche oder um eine Chromatographiesäule handelt.

8. Behälter nach Anspruch 1, **dadurch gekennzeichnet, dass** dieser keinen zusätzlichen Entlüftungsstutzen aufweist.

9. Behälter nach Anspruch 1, **dadurch gekennzeichnet, dass** dieser eine totraumarme bzw. totraumfreie Auslassvorrichtung aufweist.

10. Verwendung der in den Behälter nach Anspruch 1 angebrachten Eindüsungsund/oder Entgasungsvorrichtung zum Entgasen und/oder Eindüsen von Flüssigkeiten, insbesondere von Reinigungsflüssigkeiten, von Reaktanten oder von zu trennenden Stoffgemischen, in den Behälter.

11. Verwendung nach Anspruch 10, **dadurch gekennzeichnet, dass** der Behälter reinigbar und/oder sterilisierbar ist.
